(19) **Europäisches Patentamt**
**European Patent Office**
**Office européen des brevets**

(11) **EP 4 762 942 A1**

(12) **EUROPEAN PATENT APPLICATION**
published in accordance with Art. 153(4) EPC

(43) Date of publication:
24.06.2026 Bulletin 2026/26

(21) Application number: 24854237.5

(22) Date of filing: 16.08.2024

(51) International Patent Classification (IPC):
*A23L 2/00* (2006.01)    *A23L 2/40* (2006.01)
*A23L 5/00* (2016.01)    *A23L 33/17* (2016.01)
*C12N 9/10* (2006.01)    *C12N 9/26* (2006.01)

(52) Cooperative Patent Classification (CPC):
A23L 2/00; A23L 2/40; A23L 5/00; A23L 33/17;
C12N 9/10; C12N 9/2408

(86) International application number:
**PCT/JP2024/029202**

(87) International publication number:
**WO 2025/037649 (20.02.2025 Gazette 2025/08)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH MA MD TN**

(30) Priority: 17.08.2023  JP 2023133154

(71) Applicant: Amano Enzyme Inc.
Nagoya-shi
Aichi 460-8630 (JP)

(72) Inventor: SAKAI Kiyota
Kakamigahara-shi, Gifu 509-0109 (JP)

(74) Representative: Mullholland, Lewis Paul
WP Thompson
44 Southampton Buildings
London WC2A 1AP (GB)

(54) **METHOD FOR IMPROVING FOAMABILITY OF PLANT-BASED FOODS AND BEVERAGES**

(57)    [Summary]
[Problems] To provide a technique for improving the foamability of plant-based foods and drinks.

[Solution] The present technique provides a method for improving the foamability of plant-based foods and drinks, which includes a cyclodextrin-generating enzyme treatment step of treating a plant-based raw material with a cyclodextrin-generating enzyme, and a protein deamidating enzyme treatment step of treating the plant-based raw material with a protein deamidating enzyme.

EP 4 762 942 A1

## Description

Technical Field

The present technique relates to a method for improving the foamability of plant-based foods and drinks. More specifically, the present technique relates to a method for improving the foamability of plant-based foods and drinks, an agent for improving the foamability of plant-based foods and drinks, and plant-based foods and drinks.

Background Art

In recent years, plant-based foods and drinks have been attracting attention due to growing health consciousness. For example, an oat drink is prepared by suspending oat material in an aqueous medium and using one or more amylases to break down the starch in the oat material. The amylase used here is β-amylase or a mixture of α-amylase and β-amylase. The oat drink prepared by this method has a moderate sweetness due to the generation of maltose by the action of β-amylase (Patent Literature 1). Furthermore, because plant-based milks such as soy milk have inferior foaming stability to cow's milk, techniques using various additives are being investigated (Patent Literature 2).
Here, the enzyme used in this technique will be described. A cyclodextrin-generating enzyme is an enzyme that is used to treat starch to generate α-, β-, and γ-cyclodextrins. It is also known that a cyclodextrin-generating enzyme is used to treat starch and capable of producing cyclodextrin without liquefying starch granules (Patent Literature 3).

Citation List

Patent Literature

   Patent Literature 1: Japanese Patent Application Publication Laid-Open No. 2018-075029
   Patent Literature 2: Japanese Patent Application Publication Laid-Open No. 2020-054379
   Patent Literature 3: Japanese Patent Application Publication Laid-Open No. H09-262091

Summary of Invention

Technical Problem

As mentioned above, plant-based foods and drinks that have been attracting attention in recent years have inferior foamability compared to milk, and there is a demand for improved foamability.
Therefore, the main object of this technique is to provide a technique for improving the foamability of plant-based foods and drinks.

Solution to Problem

As a result of intensive research into techniques for improving the foamability of plant-based foods and drinks, the inventors of the present application succeeded in improving the foamability of plant-based foods and drinks by treating plant-based raw materials that are the raw materials for plant-based foods and drinks with a cyclodextrin-generating enzyme and a protein deamidating enzyme, and thus completed this technique.
That is, the present technique first provides a method for improving the foamability of plant-based foods and drinks, which includes a cyclodextrin-generating enzyme treatment step of treating a composition containing a plant-based raw material with a cyclodextrin-generating enzyme, and a protein deamidating enzyme treatment step of treating the composition comprising a plant-based raw material with a protein deamidating enzyme.
In the method for improving the foamability of plant-based foods and drinks according to the present technique, an α-amylase treatment step of treating a composition containing a plant-based raw material with α-amylase may be carried out.
Furthermore, in the method for improving the foamability of plant-based foods and drinks according to the present technique, a fat and oil addition step of adding fat and oil may be carried out.
The present technique further provides an agent for improving the foamability of plant-based foods and drinks, which comprises a cyclodextrin-generating enzyme and a protein deamidating enzyme.
Additionally, the present technique provides plant-based foods and drinks in which the agent for improving foamability according to the present technique is used.

Advantageous Effects of Invention

According to the present technique, the foamability of plant-based foods and drinks can be improved.

Description of Embodiments

A preferred embodiment for carrying out the present technique will be described below. It should be noted that the embodiment described below is an example of a typical embodiment of the present technique, and the scope of the present technique should not be interpreted as being narrowed.

1. Method for improving foamability of plant-based foods and drinks

The method for improving the foamability of plant-based foods and drinks according to the present technique is a method including a step of treating a plant-based raw material with a cyclodextrin-generating enzyme (hereinafter also referred to as a "cyclodextrin-generating enzyme treatment step") and a step of treating the plant-based raw material with a protein deamidating enzyme (hereinafter also referred to as a "protein deamidating enzyme treatment step"). The method for improving the foamability of plant-based foods and drinks according to the present technique preferably further comprises a step of treating with $\alpha$-amylase (hereinafter also referred to as the "$\alpha$-amylase treatment step"). In addition, the method for improving the foamability of plant-based foods and drinks according to the present technique preferably further comprises a fat and oil addition step of adding fat and oil. In addition, depending on the type of plant-based foods and drinks, it is also possible to carry out general food producing steps before, after, or simultaneously with each step, as long as the effects of the present technique are not impaired. Furthermore, the method for improving the foamability of plant-based foods and drinks may further carry out a recovery step of recovering the plant-based foods and drinks with improved foamability. The raw materials used and each step will be described in detail below.

(1) Plant-based raw materials

The plant-based raw materials that can be used in the present technique are not particularly limited in terms of origin, type, etc., as long as they do not impair the effects of the present technique, and can be freely selected depending on the desired plant-based foods and drinks. Examples thereof include beans such as soybeans, green peas, lentils, chickpeas, black beans, broad beans, mung beans, lupin beans, and kidney beans; grains such as wheat, barley, oats, rice, rye, buckwheat, barnyard millet, foxtail millet, and teff; nuts such as almonds, coconuts, peanuts, cashew nuts, hazelnuts, pecan nuts, macadamia nuts, pistachios, walnuts, Brazil nuts, pili nuts, chestnuts, sesame seeds, and pine nuts; and hemp seeds (industrial hemp), chia seeds, quinoa, amaranthus, canary seeds, and flaxseed. In the present technique, these may be used alone or in combination of two or more. Among these raw materials, preferred are grains, and more preferred are oats.
In the present technique, the state of the plant-based raw material when subjected to various enzyme treatments is not particularly limited as long as it does not impair the effects of the present technique, but preferably, the plant-based raw material is in a liquid or slurry state.
The plant-based raw materials that can be used in the present technique contain carbohydrates. The content of plant-based carbohydrates contained in the plant-based raw material (based on the weight of the plant-based raw material in a dry state) is not particularly limited, but may be, for example, 20% by weight or more. The content is preferably 30% by weight or more, more preferably 40% by weight or more, and even more preferably 50% by weight or more. The upper limit of the content range is not particularly limited, but examples include 90% by weight or less, 80% by weight or less, and 70% by weight or less.

(2) Cyclodextrin-generating enzyme treatment step

The cyclodextrin-generating enzyme treatment step is a step of treating a plant-based raw material with a cyclodextrin-generating enzyme. In the cyclodextrin-generating enzyme treatment step, it is preferable to treat a plant-based raw material with a cyclodextrin-generating enzyme alone, but one or more other enzymes that can be used in the production of plant-based foods and drinks can be selected and used in combination, as long as they do not affect the amount of cyclodextrin generated.
The cyclodextrin-generating enzyme that can be used in the present technique is an enzyme that has the activity to generate cyclodextrin with a degree of polymerization of 6 to 8 using polysaccharides, such as starch, that have a structure in which glucose is linked in an $\alpha$-1,4 bond as a substrate. An example of a cyclodextrin-generating enzyme is cyclodextrin glucanotransferase (EC 2.4.1.19). The cyclodextrin-generating enzyme that can be used in the present technique may be an enzyme that also has other actions, as long as it has the activity of generating cyclodextrin.

Preferably, the activity of producing cyclodextrin is the main activity.

In this technique, the foamability of a plant-based raw material can be improved by treating the plant-based raw material with a cyclodextrin-generating enzyme.

The origin of the cyclodextrin-generating enzyme that can be used in the present technique is not particularly limited, and examples thereof include cyclodextrin-generating enzymes derived from the genus Bacillus, such as Bacillus stearothermophilus, Bacillus megaterium, Bacillus circulans, Bacillus macerans, Bacillus ohbensis, and Bacillus clarkii; the genus Geobacillus, such as Geobacillus stearothermophilus; the genus Paenibacillus, such as Paenibacillus macerans; the genus Klebsiella, such as Klebsiella pneumoniae; the genus Anoxybacillus, such as Anoxybacillus caldiproteolyticus, Anoxybacillus pushchinoensis, Anoxybacillus flavithermus; the genus Thermoanaerobacter; and the genus Brevibacterium. These cyclodextrin-generating enzymes may be used alone or in combination of two or more. Among these, the cyclodextrin-generating enzyme is preferably cyclodextrin glucanotransferase derived from a microorganism of the genus Paenibacillus and/or cyclodextrin glucanotransferase derived from a microorganism of the genus Anoxybacillus, and more preferably, cyclodextrin glucanotransferase derived from Paenibacillus macerans and/or cyclodextrin glucanotransferase derived from Anoxybacillus caldiproteolyticus (including the strain formerly known as Geobacillus stearothermophilus).

Here, "cyclodextrin-generating enzyme derived from Paenibacillus macerans" and/or "cyclodextrin-generating enzyme derived from Anoxybacillus caldiproteolyticus" means a cyclodextrin-generating enzyme produced by a microorganism (which may be a wild-type strain or a mutant strain) classified as Paenibacillus macerans and/or Geobacillus stearothermophilus, or a cyclodextrin-generating enzyme obtained by genetic engineering techniques using a cyclodextrin-generating enzyme gene. Therefore, recombinant enzymes generated by a host microorganism into which a cyclodextrin-generating enzyme gene (or a modified version of said gene) obtained from Paenibacillus macerans and/or Anoxybacillus caldiproteolyticus has been introduced also fall under the category of "cyclodextrin-generating enzyme derived from Paenibacillus macerans" and/or "cyclodextrin-generating enzyme derived from Anoxybacillus caldiproteolyticus."

The cyclodextrin-generating enzyme used in the present technique can be prepared from the culture broth of the microorganism from which the above-mentioned cyclodextrin-generating enzyme is derived. A specific preparation method includes recovering the cyclodextrin-generating enzyme from the culture broth or cell bodies of the above-mentioned microorganism. For example, when a microorganism secreting a cyclodextrin-generating enzyme is used, the cell bodies may be recovered from the culture broth in advance by filtration, centrifugation, or the like, if necessary, and the enzyme may then be separated and/or purified. When a microorganism not secreting a cyclodextrin-generating enzyme is used, the cell bodies may be recovered from the culture broth in advance, if necessary, and then the cell bodies may be disrupted by pressure treatment, ultrasonic treatment, or the like to extract the enzyme, which may then be separated and/or purified. As a method for separating and/or purifying the enzyme, any known protein separation and/or purification method can be used without any particular limitation, and examples thereof include centrifugation, UF concentration, salting out, and various chromatographic methods using ion exchange resins, etc. The separated and/or purified enzyme can be powdered by a drying method such as freeze-drying or vacuum drying, and can also be powdered using an appropriate excipient and/or drying aid in the drying method. The separated and/or purified enzyme can also be formulated as a liquid by adding an appropriate additive and sterilizing by filtration.

In the present technique, commercially available cyclodextrin-generating enzymes can also be used, and preferred examples of commercially available products include cyclodextrin glucanotransferase derived from Paenibacillus macerans, manufactured by Amano Enzyme Inc., and cyclodextrin glucanotransferase derived from Anoxybacillus caldiproteolyticus (Geobacillus stearothermophilus), manufactured by Amano Enzyme Inc.

The amount of cyclodextrin-generating enzyme added in the cyclodextrin-generating enzyme treatment step can be freely set as long as it does not impair the effects of the present technique. The lower limit of the amount of cyclodextrin-generating enzyme to be added can be set to, for example, 0.01 U or more per 1 g of plant-based raw material, and from the viewpoint of further enhancing the foamability of plant-based foods and drinks, it can be set to preferably 0.03 U or more, more preferably 0.1 U or more, even more preferably 0.3 U or more, and even more preferably 1 U or more, 3 U or more, 5 U or more, or 7 U or more.

The lower limit of the amount of cyclodextrin-generating enzyme to be added can be set to, for example, 0.02 U or more per 1 g of polysaccharide (preferably starch) contained in the plant-based raw material, and from the viewpoint of further enhancing the foamability of plant-based foods and drinks, the amount can be set to preferably 0.05 U or more, more preferably 0.5 U or more, even more preferably 2 U or more, and even more preferably 5 U or more, or 10 U or more.

The upper limit of the amount of cyclodextrin-generating enzyme to be added is not particularly limited as long as it does not impair the effects of the present technique, but can be set to, for example, 200 U or less, 100 U or less, 50 U or less, 20 U or less, or 10 U or less per 1 g of plant-based raw material.

The upper limit of the amount of cyclodextrin-generating enzyme to be added can be set to, for example, 300 U or less, 200 U or less, 100 U or less, 50 U or less, 30 U or less, or 20 U or less per 1 g of polysaccharides (preferably starch) contained in the plant-based raw material.

In the present technique, the activity of the cyclodextrin-generating enzyme is a value measured by the measurement method described in the Examples below.

Various conditions for the cyclodextrin-generating enzyme treatment step can be freely set as long as they do not impair the effects of the present technique. For example, the pH, temperature, treatment time, etc. can be set depending on the physicochemical properties of the cyclodextrin-generating enzyme used, such as optimum pH, stable pH range, optimum temperature, and temperature stability. The pH can be set, for example, to pH 5.0 to 8.0, preferably pH 5.5 to 7.5, and more preferably pH 5.5 to 7.0. The temperature can be set to, for example, 30°C to 90°C, preferably 40°C to 80°C, and more preferably 50°C to 70°C. The treatment time can be set, for example, to 5 minutes to 12 hours, preferably 10 minutes to 6 hours, and more preferably 20 minutes to 120 minutes, in terms of total reaction time. The optimum reaction conditions can be determined through preliminary experiments.

(3) Protein deamidating enzyme treatment step

The protein deamidating enzyme treatment step is a step of treating a plant-based raw material with a protein deamidating enzyme. In this technique, the foamability of the produced plant-based foods and drinks can be further improved by treating a plant-based raw material with a protein deamidating enzyme.

The timing of the protein deamidating enzyme treatment step is not particularly limited, and it may be carried out before or after the cyclodextrin-generating enzyme treatment step. This step may be carried out simultaneously with the cyclodextrin-generating enzyme treatment step. It may also be carried out in several separate steps. After the cyclodextrin-generating enzyme treatment step has been performed, it is preferable to add a protein deamidating enzyme without inactivating the cyclodextrin-generating enzyme, thereby allowing the protein deamidating enzyme treatment step to proceed.

Protein deamidating enzyme that can be used in the present technique is an enzyme that exhibits the action of degrading amide group-containing side chains of proteins without cleaving peptide bonds or cross-linking proteins. The protein deamidating enzyme that can be used in the present technique may be an enzyme that also has other actions, as long as it has protein deamidating enzyme activity. It is preferable to use an enzyme whose main activity is protein deamidating enzyme activity.

Examples of protein deamidating enzymes include enzymes that deamidate glutamine residues in proteins and convert them to glutamic acid (e.g., protein glutaminase), and enzymes that deamidate asparagine residues in proteins and convert them to aspartic acid (e.g., protein asparaginase). These protein deamidating enzymes may be used singly or in combination of two or more. Among these protein deamidating enzymes, protein glutaminase is preferred from the viewpoint of further enhancing the foamability improving effect.

The origin of protein deamidating enzyme that can be used in the present technique is not particularly limited, and examples thereof include protein deamidating enzymes derived from the genera Chryseobacterium, Flavobacterium, Empedobacter, Sphingobacterium, Aureobacterium, Myroides, Luteimicrobium, Agromyces, Microbacterium, or Leifsonia. These protein deamidating enzymes are known, and reference can be made to, for example, JP2000-50887A, JP2001-218590A, WO2006/075772A1, WO2015/133590, etc.

Protein deamidating enzyme that can be used in the present technique can be prepared from the culture broth of the microorganism from which the above-mentioned protein deamidating enzyme is derived. Specific preparation methods include culturing a protein deamidating enzyme-producing bacterium and separating the protein deamidating enzyme by known means, and methods using genetic recombination technology, which can easily prepare the protein deamidating enzyme.

In the present technique, a commercially available protein deamidating enzyme can also be used, and a preferred example of a commercially available protein deamidating enzyme is protein glutaminase (derived from the species Chryseobacterium proteolyticum) manufactured by Amano Enzyme Inc.

The amount of protein deamidating enzyme added in the protein deamidating enzyme treatment step can be freely set as long as it does not impair the effects of the present technique. In the present technique, the specific lower limit of the amount of protein deamidating enzyme to be added can be set to, for example, 0.01 U or more, preferably 0.1 U or more, 0.5 U or more, and more preferably 1 U or more per 1 g of plant-based raw material.

The lower limit of the amount of protein deamidating enzyme to be added can be set to, for example, 0.05 U or more, preferably 0.2 U or more, 0.5 U or more, or 1 U or more, more preferably 3 U or more, 5 U or more, or 7 U or more, relative to 1 g of protein contained in the plant-based raw material.

The upper limit of the amount of protein deamidating enzyme to be added is not particularly limited as long as it does not impair the effects of the present technique, and can be set to, for example, 1000 U or less, 200 U or less, 100 U or less, preferably 50 U or less, 20 U or less, more preferably 10 U or less, and even more preferably 5 U or less, 3 U or less, or 2 U or less per 1 g of plant-based raw material.

The upper limit of the content of protein deamidating enzyme can be set to, for example, 2000 U or less, 400 U or less, 200 U or less, preferably 100 U or less, more preferably 50 U or less, and even more preferably 30 U or less, 20 U or less, or 15 U or less, per 1 g of protein contained in the plant-based raw material.

In the present technique, the activity of protein deamidating enzyme is a value measured by the measurement method described in the Examples below.

Various conditions for the protein deamidating enzyme treatment step can be freely set as long as they do not impair the effects of the present technique. For example, the pH, temperature, treatment time, etc. can be set depending on the physicochemical properties of the protein deamidating enzyme used, such as the optimum pH, stable pH range, optimum temperature, and temperature stability. The pH can be set, for example, to pH 5.0 to 8.0, preferably pH 5.5 to 7.5, and more preferably pH 6.0 to 7.0. The temperature can be set to, for example, 30°C to 90°C, preferably 35°C to 80°C, and more preferably 40°C to 70°C. The treatment time can be set to, for example, 5 minutes to 12 hours, preferably 10 minutes to 6 hours, and more preferably 20 minutes to 180 minutes. The optimum reaction conditions can be determined through preliminary experiments.

(4) $\alpha$-amylase treatment step

The $\alpha$-amylase treatment step is a step of treating a plant-based raw material with $\alpha$-amylase. In this technique, the viscosity of the produced plant-based foods and drinks can be reduced by treating a plant-based raw material with $\alpha$-amylase.

The timing of the $\alpha$-amylase treatment step is not particularly limited, and it may be carried out before or after the cyclodextrin-generating enzyme treatment step. This step may be carried out simultaneously with the cyclodextrin-generating enzyme treatment step. In addition, it may be carried out before or after the protein deamidating enzyme treatment step. This may be carried out simultaneously with the protein deamidating enzyme treatment step. Furthermore, it may be carried out in several separate steps. After the cyclodextrin-generating enzyme treatment step has been performed, it is preferable to proceed with the protein deamidating enzyme treatment step without inactivating the cyclodextrin-generating enzyme, and at the same time, to proceed with the $\alpha$-amylase treatment step by adding $\alpha$-amylase.

The $\alpha$-amylase that can be used in this technique is an enzyme that treats starch and mainly hydrolyzes $\alpha$-1,4 glycosidic bonds. The $\alpha$-amylase that can be used in the present technique may be an enzyme that also has other actions, as long as it has $\alpha$-amylase activity. It is preferred to use an enzyme whose predominant activity is $\alpha$-amylase activity.

The origin of the $\alpha$-amylase that can be used in the present technique is not particularly limited, and examples thereof include $\alpha$-amylases derived from organisms of the genus Aspergillus (e.g., Aspergillus oryzae, Aspergillus niger, etc.) and the genus Bacillus (e.g., Bacillus amyloliquefaciens, Bacillus subtilis, Bacillus licheniformis, etc.), preferably $\alpha$-amylases derived from the genus Bacillus or $\alpha$-amylases derived from Aspergillus, more preferably $\alpha$-amylases derived from Bacillus amyloliquefaciens or $\alpha$-amylases derived from Bacillus licheniformis.

The $\alpha$-amylase that can be used in this technique can be prepared from the culture broth of the microorganism from which the above-mentioned $\alpha$-amylase is derived. As a specific preparation method, it can be easily prepared by culturing $\alpha$-amylase-producing microorganism and separating the $\alpha$-amylase by known means, or by a method using genetic recombination technique.

In the present technique, commercially available $\alpha$-amylase can also be used, and a preferred example of a commercially available $\alpha$-amylase is $\alpha$-amylase (derived from Bacillus amyloliquefaciens) manufactured by Amano Enzyme Inc.

The amount of $\alpha$-amylase added in the $\alpha$-amylase treatment step can be freely set as long as it does not impair the effects of the present technique. In the present technique, the specific lower limit of the amount of $\alpha$-amylase to be added can be set to, for example, 0.01 U or more per 1 g of plant-based raw material, preferably 0.1 U or more, 0.5 U or more, 1 U or more, more preferably 2 U or more, 3 U or more.

The lower limit of the amount of $\alpha$-amylase to be added can be set to, for example, 0.05 U or more, preferably 0.2 U or more, 0.5 U or more, or 1 U or more, more preferably 2 U or more, 3 U or more, or 4 U or more, per 1 g of polysaccharides (preferably starch) contained in the plant-based raw material.

The upper limit of the amount of $\alpha$-amylase to be added is not particularly limited as long as it does not impair the effects of the present technique, and can be set to, for example, 1000 U or less, 200 U or less, 100 U or less, preferably 50 U or less, 20 U or less, more preferably 10 U or less, and even more preferably 5 U or less, 3 U or less, or 1 U or less per 1 g of plant-based raw material.

The upper limit of the $\alpha$-amylase content can be set to, for example, 2000 U or less, 400 U or less, 200 U or less, preferably 100 U or less, more preferably 30 U or less, and even more preferably 10 U or less, 4 U or less, or 2 U or less per 1 g of polysaccharides (preferably starch) contained in the plant-based raw material.

In the present technique, the $\alpha$-amylase activity is a value measured by the measurement method described in the Examples below.

Various conditions for the $\alpha$-amylase treatment step can be freely set as long as they do not impair the effects of the present technique. For example, the pH, temperature, treatment time, etc. can be set depending on the physicochemical properties of the α-amylase used, such as the optimum pH, stable pH range, optimum temperature, and temperature stability. The pH can be set, for example, to pH 5.0 to 8.0, preferably pH 5.5 to 7.5, and more preferably pH 6.0 to 7.0. The temperature can be set to, for example, 30°C to 90°C, preferably 35°C to 80°C, and more preferably 40°C to 70°C. The treatment time can be set to, for example, 5 minutes to 12 hours, preferably 10 minutes to 6 hours, and more preferably 20 minutes to 180 minutes. The optimum reaction conditions can be determined through preliminary experiments.

(5) Fat and oil addition step

The fat and oil adding step is a step of adding fat and oil. The fat and oil addition step also includes using a raw material containing fat and oil as the plant-based raw material. The fat and oil to be added may be of the same origin as the plant-based raw material or may be of a different origin. By including fat and oil, foamability can be further improved. The origin, type, etc. of the fat and oil are not particularly limited and can be freely selected depending on the intended plant-based foods and drinks. Examples thereof include soybean oil, canola oil, sunflower oil, corn oil, olive oil, rice oil, palm oil, safflower oil, cottonseed oil, and the like.

The timing of the fat and oil addition step is not particularly limited, and it may be carried out before or after the cyclodextrin-generating enzyme treatment step. This step may be carried out simultaneously with the cyclodextrin-generating enzyme treatment step. In addition, it may be carried out before or after the protein deamidating enzyme treatment step. This may be carried out simultaneously with the protein deamidating enzyme treatment step. Furthermore, when the α-amylase treatment step is carried out, it may be carried out before or after the α-amylase treatment step. This may be carried out simultaneously with the α-amylase treatment step.

(6) Recovery step

The recovery step is a step of recovering plant-based foods and drinks with improved foamability that have been produced through the cyclodextrin-generating enzyme treatment step and the protein deamidating enzyme treatment step. As for the specific recovery method, one or a combination of two or more recovery methods generally used in the production of plant-based foods and drinks can be used freely depending on the type of plant-based foods and drinks to be produced.

The plant-based foods and drinks produced through the cyclodextrin-generating enzyme treatment step and the protein deamidating enzyme treatment step is characterized by improved foamability. Specifically, for example, the foamability of plant-based foods and drinks produced through a cyclodextrin-generating enzyme treatment step and a protein deamidating enzyme treatment step is improved by 10% or more, preferably 20% or more, and more preferably 30% or more, compared to plant-based foods and drinks produced in the same manner except that the cyclodextrin-generating enzyme treatment step and the protein deamidating enzyme treatment step are not performed. That is, the present technique can carry out a recovery step of recovering plant-based foods and drinks with foamability improved by 10% or more, preferably 20% or more, and more preferably 30% or more.

One embodiment of the method for improving the foamability of plant-based foods and drinks according to the present technique includes the following steps (1) to (3). In addition, a step (4) of treating with α-amylase may be carried out before, after, or simultaneously with step (2) or (3). Furthermore, after steps (2) to (4), a step (5) of inactivating the enzyme may be added. Before or after steps (2) to (5), step (6) of adding fat and oil may be added. After steps (2) to (6), a step (7) of recovering the plant-based foods and drinks with improved foamability may be added.

(1) A step of preparing a raw material containing plant-based carbohydrates
(2) A step of treating the prepared raw material with a cyclodextrin-generating enzyme
(3) A step of treating the prepared raw material with protein deamidating enzyme

One embodiment of the method for improving the foamability of plant-based foods and drinks according to the present technique includes the following steps (1) to (3). Furthermore, after step (3), the following steps may be added: (4) a step of inactivating the enzyme, (5) a step of adding fat and oil, and/or (6) a step of recovering the plant-based foods and drinks with improved foamability. The timing of the enzyme inactivation step (4) is not limited as long as it is performed after step (3). The timing of the fat and oil addition step (5) is not limited as long as it is performed after step (1).

(1) A step of preparing a raw material containing plant-based carbohydrates and fat and oil
(2) A step of treating the prepared raw material with a cyclodextrin-generating enzyme
(3) A step of adding protein deamidating enzyme and α-amylase to the raw material being treated with the cyclodextrin-generating enzyme while continuing the step (2), and treating the raw material with the protein deamidating enzyme and α-amylase.

In the step (6), plant-based foods and drinks having foamability improved by 10% or more can also be recovered.

2. Agent for improving the foamability of plant-based foods and drinks

The agent for improving the foamability of plant-based foods and drinks according to the present technique contains a cyclodextrin-generating enzyme and a protein deamidating enzyme. For example, by adding an agent for improving foamability containing a cyclodextrin-generating enzyme and a protein deamidating enzyme to the plant-based raw material used in the production of plant-based foods and drinks, the cyclodextrin-generating enzyme treatment step and the protein deamidating enzyme treatment step can be progressed, and as described above, the foamability in plant-based foods and drinks to be produced can be improved. The constitution of the agent for improving foamability will be described in detail below.

(1) Cyclodextrin-generating enzyme

The agent for improving the foamability of plant-based foods and drinks according to the present technique contains a cyclodextrin-generating enzyme. The details of the cyclodextrin-generating enzyme are the same as those described for the method for improving the foamability of plant-based foods and drinks, and therefore will not be described here.

The content of the cyclodextrin-generating enzyme in the agent for improving the foamability of plant-based foods and drinks according to the present technique can be freely set as long as it does not impair the effects of the present technique. The content of the cyclodextrin-generating enzyme is the same as the amount of the cyclodextrin-generating enzyme added in the above-mentioned cyclodextrin-generating enzyme treatment step, and therefore, a detailed description thereof will be omitted here.

(2) Protein deamidating enzyme

The agent for improving the foamability of plant-based foods and drinks according to the present technique contains protein deamidating enzyme. The details of the protein deamidating enzyme are the same as those described for the method for improving the foamability of plant-based foods and drinks, and therefore will not be described here.

The content of the protein deamidating enzyme in the agent for improving the foamability of plant-based foods and drinks according to the present technique can be freely set as long as it does not impair the effects of the present technique. The content of protein deamidating enzyme is the same as the amount of protein deamidating enzyme added in the above-mentioned protein deamidating enzyme treatment step, and therefore, description thereof will be omitted here.

(3) $\alpha$-amylase

The agent for improving the foamability of plant-based foods and drinks according to the present technique may also contain $\alpha$-amylase. By using $\alpha$-amylase, the viscosity of plant-based foods and drinks can be reduced. The details of the $\alpha$-amylase are the same as those described for the method for improving the foamability of plant-based foods and drinks, and therefore will not be described here.

The content of $\alpha$-amylase in the agent for improving the foamability of plant-based foods and drinks according to the present technique can be freely set as long as it does not impair the effects of the present technique. The content of $\alpha$-amylase is the same as the amount of $\alpha$-amylase added in the above-mentioned $\alpha$-amylase treatment step, and therefore, a detailed explanation is omitted here.

The agent for improving the foamability of plant-based foods and drinks according to the present technique preferably comprises a first agent containing a cyclodextrin-generating enzyme, a second agent containing a protein deamidating enzyme, and a third agent containing $\alpha$-amylase. By preparing the cyclodextrin-generating enzyme, protein deamidating enzyme, and $\alpha$-amylase as separate agents, the order, etc. in which the composition containing the plant-based raw material is treated with them can be easily adjusted to suit the purpose.

In this case, the first agent may contain only the cyclodextrin-generating enzyme alone. That is, the first agent may be the cyclodextrin-generating enzyme itself. The second agent may contain only protein deamidating enzyme alone. That is, the second agent may be protein deamidating enzyme itself. Furthermore, the second agent may contain a cyclodextrin-generating enzyme in addition to the protein deamidating enzyme. The third agent may contain only $\alpha$-amylase. That is, the third agent may be $\alpha$-amylase itself. Furthermore, the third agent may contain, in addition to $\alpha$-amylase, a cyclodextrin-generating enzyme and/or a protein deamidating enzyme. In addition, the first, second, and third agents may contain one or more selected other enzymes that can be used in the production of plant-based foods and drinks, as long as the addition does not affect the improvement of foamability.

(4) Other

The agent for improving the foamability of plant-based foods and drinks according to the present technique can also freely contain one or more other components, as long as the effects of the present technique are not impaired. Examples of other components that can be used include excipients, pH adjusters, colorants, flavoring agents, disintegrants, lubricants, stabilizers, and other components that are commonly used in formulations. Furthermore, components having known or future functions can also be used in combination depending on the purpose.

In addition, the agent for improving the foamability of plant-based foods and drinks according to the present technique may further contain a fourth agent, a fifth agent, etc. that contain one or more selected from other enzymes that can be used in the production of plant-based foods and drinks, and components with known or future functions, in addition to the first, second, and third agents described above, as long as the effect of improving foamability is not affected.

3. Plant-based foods and drinks

The plant-based foods and drinks according to the present technique are plant-based foods and drinks produced using the agent for improving foamability described above. Specific examples of plant-based drinks include oat drinks, and more specifically oat milk (also known as "oats milk").

The plant-based foods and drinks according to the present technique has improved foamability. The foamability of plant-based foods and drinks according to the present technique is not particularly limited. The foamability of plant-based foods and drinks is improved by 10% or more, preferably 20% or more, and more preferably 30% or more compared to plant-based foods and drinks produced in the same manner except that no agent for improving foamability is used.

EXAMPLES

The present invention will be described in more detail below based on examples. The examples described below are merely representative examples of the present invention, and the scope of the present invention should not be construed as being narrowed.

1. Enzymes used

Cyclodextrin-generating enzyme (CGT): Cyclodextrin glucanotransferase derived from Anoxybacillus caldiproteolyticus (formerly known as Geobacillus stearothermophilus), manufactured by Amano Enzyme Inc.

Protein deamidating enzyme (PG): Protein glutaminase derived from Chryseobacterium proteolyticum, manufactured by Amano Enzyme Inc.

α-Amylase (AMY): α-amylase derived from Bacillus amyloliquefaciens, manufactured by Amano Enzyme Inc.

2. Enzyme activity measurement method

[Method for measuring cyclodextrin-generating enzyme activity]

The activity of the cyclodextrin-generating enzyme can be measured by a method based on the cyclodextrin-generating enzyme activity test method in the 9th edition of the Japan's Specifications and Standards for Food Additives.

20 mL of water was added to 1.0 g of potato starch, and 5 mL of sodium hydroxide reagent solution (1 mol/L) was gradually added while stirring to form a paste. After heating in a boiling water bath with stirring for 3 minutes, 25 mL of water was added, cooled with running water, adjusted to pH 5.5 with acetic acid reagent solution (1 mol/L), and further added with water to make 100 mL, which was used as the substrate solution. 10 mL of the substrate solution was measured out and warmed at 40°C for 10 minutes, and 1 mL of the enzyme solution was added and mixed. After incubation at 40°C for 10 minutes, the reaction was stopped by adding 10 mL of hydrochloric acid reagent solution (0.1 mol/L). To 1 mL of this reaction solution, 10 mL of iodine-potassium iodide reagent solution (0.4 mmol/L) was added and mixed to prepare a test solution. The iodine/potassium iodide reagent solution was prepared by dissolving 10.0 g of potassium iodide and 1.0 g of iodine in water to make 100 mL, and then diluting 200 times with water. A control solution was prepared by using water instead of the reaction solution, and the absorbance at 660 nm of the test solution and the control solution was measured. The amount of enzyme that reduces the blue iodine color of starch by 1% in 1 minute was defined as 1 unit (U).

[Method for measuring protein deamidating enzyme activity]

0.1 mL of the sample solution containing protein deamidating enzyme was added to 1 mL of 0.2 M phosphate buffer (pH 6.5) containing 30 mM Z-Gln-Gly, and the mixture was allowed to stand at 37°C for 10 minutes, after which 1 mL of 0.4 M TCA solution was added to stop the reaction. As a blank, 1 mL of 0.4 M TCA solution was added to 1 mL of 0.2 M phosphate buffer (pH 6.5) containing 30 mM Z-Gln-Gly, and 0.1 mL of the sample solution containing protein deamidating enzyme was further added, followed by standing at 37°C for 10 minutes.

Regarding the solution obtained as described above, the amount of ammonia generated in the reaction solution was measured using an Ammonia Test Wako (Fujifilm Wako Pure Chemical Industries, Ltd.). The ammonia concentration in the reaction solution was determined from a calibration curve showing the relationship between ammonia concentration and absorbance (630 nm) prepared using an ammonia standard solution (ammonium chloride).

The activity of protein deamidating enzyme was calculated according to the following formula, with the amount of enzyme that generates 1 μmol of ammonia in 1 minute being 1 unit (1 U). In the formula, the volume of the reaction solution is 2.1, the volume of the enzyme solution is 0.1, and Df is the dilution factor of the enzyme solution. Also, 17.03 is the molecular weight of ammonia.

protein deamidating enzyme activity (U/mL) = (concentration of ammonia in reaction solution)(mg/L) × (1/17.03) × (amount of reaction solution/amount of enzyme solution) × (1/10) × Df     [Numerical Formula 1]

[Method for measuring α-amylase activity]

10 mL of 1% potato starch substrate solution (0.1 mol/L acetic acid (pH 5.0)) was warmed at 37°C for 10 minutes, and then 1 mL of the sample solution containing α-amylase was added and immediately shaken and mixed. This solution was allowed to stand at 37°C for 10 minutes, and then 1 mL of this solution was added to 10 mL of 0.1 mol/L hydrochloric acid reagent solution, and the mixture was immediately shaken and mixed. Next, 0.5 mL of this solution was measured, and 10 mL of 0.0002 mol/L iodine reagent solution (Japanese Pharmacopoeia) was added and shaken and mixed, and then the absorbance (AT) at a wavelength of 660 nm was measured using water as a control. Separately, 1 mL of water was added instead of the sample solution, and the same procedure was repeated to measure the absorbance (AB). The 0.0002 mol/L iodine reagent solution (Japanese Pharmacopoeia) was prepared by adding 10 mL of water to 12.7 g of iodine and 25 g of potassium iodide, mixing well, adding water to make 100 mL, and then diluting 2500 times with water. The α-amylase activity was calculated using the following formula. The amount of enzyme that reduces the color of potato starch caused by iodine by 10% in 1 minute was defined as 1 unit (1 U).

$$\alpha\text{-Amylase activity (U/g, U/mL)} = \{(AB-AT)/AB\} \times 1/W$$

AT: absorbance of reaction solution
AB: absorbance of blank solution
W: Amount of sample in 1 mL of sample solution (g or mL)

3. Experimental Example 1

(1) Experimental method

18.1 g of oat flour and 150 g of water were mixed and suspended in a 300 mL Erlenmeyer flask. This suspension (oat flour content: 10.8% by weight, protein content: 1.4% by weight, dietary fiber content: 1.1% by weight, starch content: 7.3% by weight) was completely gelatinized. The enzymes shown in Table 1 were added in amounts shown in Table 1, and the mixture was reacted at 60°C for 0.5 hours, and boiled for 5 minutes at the end of the reaction. After filtering through a 100 mesh filter, canola oil was added to the obtained oat base drink to a final concentration of 2%, and the mixture was homogenized at 200 bar. The oat drink was then foamed by stirring in a milk frother at 65°C for 3 minutes. The foamability and foam stability were calculated using the following formula.

$$\text{Foamability (\%)} = (VF0/60) \times 100$$

$$\text{Foam stability (\%)} = (VF30/VF0) \times 100$$

60: Liquid volume
VF0: Volume of foam + liquid immediately after foaming
VF30: Volume of foam + liquid after leaving to stand for 30 minutes after foaming

(2) Results

The results are shown in Table 1 below.

[Table 1]

|  | CGT addition amount U/g-starch | AMY addition amount U/g-starch | foamability % | foam stability % |
|---|---|---|---|---|
| Reference Example 1 | - | 10.6 | 123 | 92 |
| Reference Example 2 | 0.65 | - | 133 | 90 |
| Reference Example 3 | 3.25 | - | 143 | 93 |
| Reference Example 4 | 6.5 | - | 152 | 91 |
| Reference Example 6 | 13 | - | 167 | 91 |

(3) Discussion

As shown in Reference Examples 2 to 5 in Table 1, it was confirmed that the foamability was improved by the cyclodextrin-generating enzyme treatment. It was also confirmed that the foam stability was the same.

4. Experimental Example 2

(1) Experimental method

18.1 g of oat flour and 150 g of water were mixed and suspended in a 300 mL Erlenmeyer flask. This suspension (oat flour content: 10.8% by weight, protein content: 1.4% by weight, dietary fiber content: 1.1% by weight, starch content: 7.3% by weight) was completely gelatinized. The enzymes shown in Table 2 were added in the amounts shown in Table 2 for the first-stage reaction (60°C, 0.5 hours) and the second-stage reaction (50°C, 2.0 hours). At the end of the reaction, the mixture was boiled for 5 minutes. After filtering through a 100 mesh filter, canola oil was added to the obtained oat base drink to a final concentration of 2%, and the mixture was homogenized at 200 bar. The oat drink was then foamed by stirring in a milk frother at 65°C for 3 minutes. The foamability and foam stability were calculated in the same manner as in Experimental Example 1.

(2) Results

The results are shown in Table 2 below.

[Table 2]

|  | first stage reaction | | second stage reaction | foamability % | foam stability % |
|---|---|---|---|---|---|
|  | CGT addition amount U/g-starch | AMY addition amount U/g-starch | AMY addition amount U/g-starch |  |  |
| Reference Example 6 | - | 10.6 | - | 123 | 91 |
| Reference Example 7 | 13 | - | 21.2 | 132 | 93 |
| Reference Example 8 | 13 | - | 10.6 | 137 | 91 |
| Reference Example 9 | 13 | - | 5.3 | 142 | 92 |
| Reference Example 10 | 13 | - | 1.06 | 143 | 91 |

(3) Discussion

As shown in Reference Examples 7 to 10 in Table 2, it was confirmed that the foamability was improved even when $\alpha$-amylase treatment was carried out after cyclodextrin-generating enzyme treatment. Furthermore, the viscosity reducing effect was also confirmed through α-amylase treatment.

5. Experimental Example 3

(1) Experimental method

18.1 g of oat flour and 150 g of water were mixed and suspended in a 300 mL Erlenmeyer flask. This suspension (oat flour content: 10.8% by weight, protein content: 1.4% by weight, dietary fiber content: 1.1% by weight, starch content: 7.3% by weight) was completely gelatinized. The enzymes shown in Table 3 were added in the amounts shown in Table 3 for the first-stage reaction (60°C, 0.5 hours) and the second-stage reaction (50°C, 2.0 hours). At the end of the reaction, the mixture was boiled for 5 minutes. After filtering through a 100 mesh filter, canola oil was added to the obtained oat base drink to a final concentration of 2%, and the mixture was homogenized at 200 bar. The mixture was then stirred in a milk frother at 65°C for 3 minutes to foam the oat drink. The foamability and foam stability were calculated in the same manner as in Experimental Example 1.

(2) Results

The results are shown in Table 3 below.

[Table 3]

| | first stage reaction | | second stage reaction | | foamability % | foam stability % |
|---|---|---|---|---|---|---|
| | CGT addition amount U/g-starch | AMY addition amount U/g-starch | AMY addition amount U/g-starch | PG addition amount U/g-protein | | |
| Reference Example 11 | - | 10.6 | - | - | 123 | 92 |
| Comparative Example 1 | - | 10.6 | - | 10 | 140 | 89 |
| Comparative Example 2 | 13 | - | 10.6 | - | 142 | 91 |
| Example 1 | 13 | - | 10.6 | 10 | 173 | 90 |

(3) Discussion

As shown in Table 3, it was found that the foamability of Comparative Examples 1 and 2, which were subjected to a cyclodextrin-generating enzyme treatment or a protein deamidating enzyme treatment, was improved compared to Reference Example 11, which was subjected to an α-amylase treatment. Furthermore, it was confirmed that the foamability was improved synergistically by using a cyclodextrin-generating enzyme and a protein deamidating enzyme in combination. Furthermore, the foam stability was the same, and it was confirmed that there was no effect on stability.

**Claims**

1.

A method for improving the foamability of plant-based foods and drinks, comprising
a cyclodextrin-generating enzyme treatment step of treating a composition comprising a plant-based raw material with a cyclodextrin-generating enzyme, and
a protein deamidating enzyme treatment step of treating the composition comprising a plant-based raw material with a protein deamidating enzyme.

2. The method for improving the foamability of plant-based foods and drinks according to claim 1, comprising an α-amylase treatment step of treating a composition containing a plant-based raw material with α-amylase.

3. The method for improving the foamability of plant-based foods and drinks according to claim 1, comprising a fat and oil addition step of adding fat and oil.

4. An agent for improving the foamability of plant-based foods and drinks, comprising a cyclodextrin-generating enzyme and a protein deamidating enzyme.

5. Plant-based foods and drinks in which the agent for improving foamability according to claim 4 is used.

# EP 4 762 942 A1

## INTERNATIONAL SEARCH REPORT

<table>
<tr><td colspan="2">International application No.</td></tr>
<tr><td colspan="2"><b>PCT/JP2024/029202</b></td></tr>
</table>

**A. CLASSIFICATION OF SUBJECT MATTER**

*A23L 2/00*(2006.01)i; *A23L 2/40*(2006.01)i; *A23L 5/00*(2016.01)i; *A23L 33/17*(2016.01)i; *C12N 9/10*(2006.01)i; *C12N 9/26*(2006.01)i

FI: A23L2/00 S; C12N9/10; A23L33/17; C12N9/26 A; A23L2/40; A23L5/00 Z; A23L5/00 J

According to International Patent Classification (IPC) or to both national classification and IPC

**B. FIELDS SEARCHED**

Minimum documentation searched (classification system followed by classification symbols)

A23L2/00; A23L2/40; A23L5/00; A23L33/17; C12N9/10; C12N9/26

Documentation searched other than minimum documentation to the extent that such documents are included in the fields searched

Published examined utility model applications of Japan 1922-1996
Published unexamined utility model applications of Japan 1971-2024
Registered utility model specifications of Japan 1996-2024
Published registered utility model applications of Japan 1994-2024

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
|---|---|---|
| X | WO 2022/097675 A1 (AMANO ENZYME INC.) 12 May 2022 (2022-05-12) claims, paragraphs [0072]-[0075], examples | 5 |
| Y | | 1-5 |
| Y | JP 2015-223163 A (NIHON SHOKUHIN KAKO CO., LTD.) 14 December 2015 (2015-12-14) claims, paragraph [0019], examples | 1-5 |
| Y | CN 112741179 A (INNER MONGOLIA YILI INDUSTRIAL GROUP CO., LTD.) 04 May 2021 (2021-05-04) claims, examples | 1-5 |
| Y | CN 115191490 A (RUIXING COFFEE INFORMATION TECH XIAMEN CO., LTD.) 18 October 2022 (2022-10-18) claims, examples | 1-5 |

✓ Further documents are listed in the continuation of Box C.    ✓ See patent family annex.

\*    Special categories of cited documents:
"A"  document defining the general state of the art which is not considered to be of particular relevance
"D"  document cited by the applicant in the international application
"E"  earlier application or patent but published on or after the international filing date
"L"  document which may throw doubts on priority claim(s) or which is cited to establish the publication date of another citation or other special reason (as specified)
"O"  document referring to an oral disclosure, use, exhibition or other means
"P"  document published prior to the international filing date but later than the priority date claimed

"T"  later document published after the international filing date or priority date and not in conflict with the application but cited to understand the principle or theory underlying the invention
"X"  document of particular relevance; the claimed invention cannot be considered novel or cannot be considered to involve an inventive step when the document is taken alone
"Y"  document of particular relevance; the claimed invention cannot be considered to involve an inventive step when the document is combined with one or more other such documents, such combination being obvious to a person skilled in the art
"&"  document member of the same patent family

| Date of the actual completion of the international search | Date of mailing of the international search report |
|---|---|
| **10 October 2024** | **22 October 2024** |

| Name and mailing address of the ISA/JP | Authorized officer |
|---|---|
| **Japan Patent Office (ISA/JP)** 3-4-3 Kasumigaseki, Chiyoda-ku, Tokyo 100-8915 Japan | |
| | Telephone No. |

Form PCT/ISA/210 (second sheet) (July 2022)

**INTERNATIONAL SEARCH REPORT**

| International application No. |
|---|
| **PCT/JP2024/029202** |

**C.    DOCUMENTS CONSIDERED TO BE RELEVANT**

| Category* | Citation of document, with indication, where appropriate, of the relevant passages | Relevant to claim No. |
|---|---|---|
| Y | JP 2001-218590 A (AMANO ENZYME INC.) 14 August 2001 (2001-08-14) claims, paragraph [0008], examples | 1-5 |

Form PCT/ISA/210 (second sheet) (July 2022)

**INTERNATIONAL SEARCH REPORT**
Information on patent family members

International application No.

**PCT/JP2024/029202**

| Patent document cited in search report | | | Publication date (day/month/year) | Patent family member(s) | | | Publication date (day/month/year) |
|---|---|---|---|---|---|---|---|
| WO | 2022/097675 | A1 | 12 May 2022 | US | 2024/0099322 | A1 | |
| | | | | claims, paragraphs [0086]-[0089], examples | | | |
| | | | | EP | 4241571 | A1 | |
| | | | | CN | 114521593 | A | |
| JP | 2015-223163 | A | 14 December 2015 | (Family: none) | | | |
| CN | 112741179 | A | 04 May 2021 | (Family: none) | | | |
| CN | 115191490 | A | 18 October 2022 | (Family: none) | | | |
| JP | 2001-218590 | A | 14 August 2001 | US | 2004/0166558 | A1 | |
| | | | | claims, paragraph [0008], examples | | | |
| | | | | EP | 1106696 | A1 | |

Form PCT/ISA/210 (patent family annex) (July 2022)

**REFERENCES CITED IN THE DESCRIPTION**

**Patent documents cited in the description**

- JP 2018075029 A
- JP 2020054379 A
- JP H09262091 A
- JP 2000050887 A
- JP 2001218590 A
- WO 2006075772 A1
- WO 2015133590 A

**Non-patent literature cited in the description**

- Japan's Specifications and Standards for Food Additives